# EUROPÄISCHE PATENTSCHRIFT

(11) **EP 0 669 330 B1**
(45) Veröffentlichungstag und Bekanntmachung des Hinweises auf die Patenterteilung: **05.08.1998**
(21) Anmeldenummer: 95810099.2
(22) Anmeldetag: 15.02.1995
(51) Int. Cl.: C07D 403/14, C08K 5/3475, C07D 403/12

(54) **Dimere Benztriazole als UV-Absorber**
Benzotriazole dimers useful as U.V. absorbers
Dimères de benzotriazole utiles comme absorbant UV

(30) Priorität: 24.02.1994 CH 554/94
(43) Veröffentlichungstag der Anmeldung: 30.08.1995
(73) Patentinhaber: Ciba Specialty Chemicals Holding Inc., 4057 Basel (CH)
(72) Erfinder: Reinehr, Dieter, Dr., D-79400 Kandern (DE); Bacher, Jean-Pierre, F-68220 Buschwiller (FR); Schmitter, André, Dr., F-68220 Hegenheim (FR)

(56) Entgegenhaltungen:
- CH-A- 408 033
- CH-A- 410 851
- CH-A- 497 919
- DE-A- 2 536 335
- US-A- 3 399 173
- US-A- 4 681 905
- US-A- 4 812 498
- US-A- 4 859 726
- US-A- 4 948 666
- US-A- 5 233 047

## Beschreibung

Die Erfindung betrifft neue dimere 2-(2 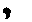 -Hydroxyphenyl)-benztriazole, deren Verwendung als Stabilisatoren für organische Polymere, entsprechende stabilisierte Zusammensetzungen und ein Verfahren zum Stabilisieren organischer Polymere.

Bestimmte 2-(2 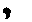 -Hydroxyphenyl)-benztriazole werden seit längerem als Stabilisatoren für organische Polymere eingesetzt. Auch die Verwendung einiger dimerer Verbindungen dieses Typs ist beschrieben. So sind einige Methylen-verbrückte Bis-benztriazole beispielsweise aus US-A-4 812 498, US-A-4 948 666 und US-A-4 681 905 bekannt;
US-A-4 859 726 beschreibt 2-(2 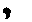 -Hydroxyphenyl)-benztriazole, die in 3 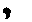 -Stellung über Di-isopropylidenbenzol chemisch aneinander gebunden sind.

DE-A-2 536 335, GB-A-1 169 859 und CH-A-408 033 beschreiben einige 2-(2 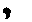 -Hydroxyphenyl)-benztriazole, die in 3'-Stellung einen Substituenten tragen vom Typ -CH₂-N(R)-CO-R, wobei R jeweils für bestimmte organische Reste steht, und die ebenfalls als Lichtschutzmittel einsetzbar sind.

Es besteht weiterhin Bedarf an dimeren Benztriazol-UV-Absorbern.

Gegenstand der Erfindung sind neue dimere Benztriazole, die sich überraschend gut zur Stabilisierung organischer Polymere gegen den schädigenden Einfluß von Wärme, Sauerstoff und Licht, insbesondere UV-Strahlung, eignen.

Die Erfindung betrifft daher Verbindungen der Formel I 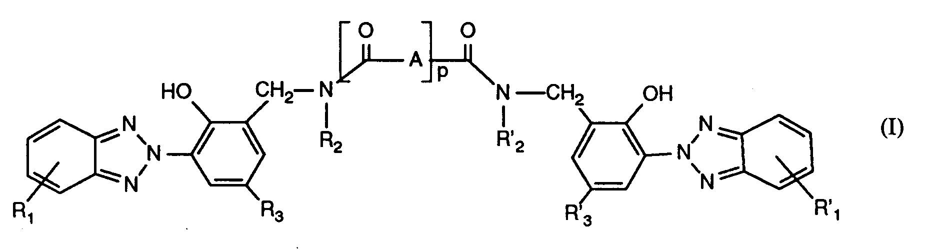
worin p 0 oder 1 ist;
A C₁-C₁₂-Alkylen darstellt;
R₁ und R 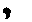 ₁ unabhängig voneinander Wasserstoff, Halogen, C₁-C₁₈-Alkyl, C₁-C₁₈-Alkoxy oder -CN bedeuten;
R₂ und R 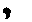 ₂ unabhängig voneinander Wasserstoff oder C₁-C₁₈-Alkyl oder gemeinsam C₂-C₁₂-Alkylen oder C₂-C₁₂-Hydroxyalkylen darstellen; und
R₃ und R 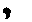 ₃ unabhängig voneinander Wasserstoff, Halogen, C₁-C₁₈-Alkyl oder C₁-C₁₈-Alkoxy bedeuten.

Es ist besonders die außerordentlich hohe Sublimationsechtheit der Verbindungen der Formel I sowie ihre gute Beständigkeit gegenüber Extraktion hervorzuheben.

Verbindungen der Formel I, worin p 0 ist, sind bevorzugt.

Weiterhin bevorzugt sind Verbindungen der Formel I, worin
R₁ und R 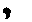 ₁ unabhängig voneinander Wasserstoff, Halogen, C₁-C₁₂-Alkyl, C₁-C₁₂-Alkoxy oder -CN bedeuten;
R₂ und R 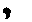 ₂ unabhängig voneinander Wasserstoff oder C₁-C₁₂-Alkyl oder gemeinsam C₂-C₃-Alkylen oder C₂-C₃-Hydroxyalkylen darstellen; und
R₃ und R 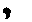 ₃ unabhängig voneinander Wasserstoff, Chlor, C₁-C₁₂-Alkyl oder C₁-C₁₂-Alkoxy bedeuten.

Von herausragendem Interesse sind Verbindungen der Formel I, worin p 0 ist und
R₁ und R 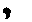 ₁ gleich sind und Wasserstoff, C₁-C₄-Alkoxy oder Chlor bedeuten;
R₂ und R 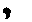 ₂ gleich sind und Wasserstoff oder C₁-C₄-Alkyl oder gemeinsam C₂-C₃-Alkylen oder C₂-C₃-Hydroxyalkylen darstellen; und
R₃ und R 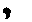 ₃ gleich sind und C₁-C₉-Alkyl oder C₁-C₄-Alkoxy bedeuten;
   darunter vor allem Verbindungen der Formel I, worin p 0 ist und
R₁ und R 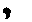 ₁ gleich sind und Wasserstoff oder Chlor bedeuten;
R₂ und R 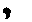 ₂ gleich sind und Wasserstoff oder Methyl bedeuten, oder R₂ und R 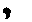 ₂ gemeinsam Ethylen oder 1,2-Dihydroxyethylen darstellen; und
R₃ und R 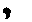 ₃ gleich sind und C₁-C₉-Alkyl bedeuten.

Halogen bedeutet -F, -Cl, -Br oder -I; in allen Ausführungsformen der Erfindung ist ein Halogensubstituent vorzugsweise -Cl oder -Br; vor allem -Cl.

A als Alkylen bedeutet Methylen, Ethylen, Propylen, Butylen, Pentylen, Hexylen, Heptylen, Octylen, Nonylen, Decylen, Undecylen oder Dodecylen. Bevorzugt ist C₁-C₈-Alkylen, insbesondere geradkettiges C₁-C₈-Alkylen.

R₁,R 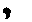 ₁,R₂,R 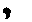 ₂,R₃ und R 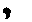 ₃ als Alkyl sind, unabhängig voneinander, im Rahmen ihrer angegebenen Bedeutungen Methyl, Ethyl, Propyl, Butyl, Pentyl, Hexyl, Heptyl, Octyl, Nonyl, Decyl, Undecyl, Dodecyl, Tridecyl, Tetradecyl, Pentadecyl, Hexadecyl, Heptadecyl oder Octadecyl; bevorzugt ist C₁-C₁₂-Alkyl, besonders C₁-C₉-Alkyl. R₁,R 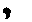 ₁,R₂ und R 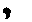 ₂ als Alkyl stellen, unabhängig voneinander, bevorzugt C₁-C₄-Alkyl, vor allem Methyl, dar.

R₃ und R 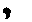 ₃ als Alkyl sind vorzugsweise, unabhängig voneinander, entweder Methyl oder verzweigtkettiges C₄-C₁₂-Alkyl, insbesondere Methyl oder tertiäres C₄-C₈-Alkyl. Eine tertiäre Alkylgruppe bezeichnet dabei einen gesättigten aliphatischen Kohlenwasserstoffrest, dessen bindendes Kohlenstoffatom seinerseits an 3 weitere Kohlenstoffatome bindet. Beispiele für tertiäres C₄-C₈-Alkyl sind unter anderen tert.-Butyl (1,1-Dimethylethyl) oder tert.-Octyl(1,1,3,3-Tetramethylbutyl).

Vorzugsweise stellen R₁ und R 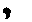 ₁ ebenso wie R₃ und R 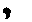 ₃ jeweils gleiche Reste dar. Auch R₂ und R 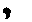 ₂ sind, sofern sie nicht gemeinsam Alkylen oder Hydroxyalkylen darstellen, gleiche Reste.

Stellen R₂ und R 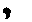 ₂ gemeinsam Alkylen oder Hydroxyalkylen dar, so ist p im allgemeinen 0; R₂ und R 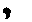 ₂ bilden dann vorzugsweise zusammen mit der Harnstoff-Einheit, an die sie gebunden sind, einen fünfgliedrigen oder sechsgliedrigen Ring, vor allem einen 5-gliedrigen Ring. Der Ring kann durch Alkyl oder, wenn R₂ und R 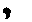 ₂ gemeinsam die Bedeutung Hydroxyalkylen haben, zusätzlich durch 1 oder mehrere -OH substituiert sein. Besonders bevorzugt stellen R₂ und R 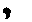 ₂ als Alkylen oder Hydroxyalkylen gemeinsam 1,2-Ethylen, 1,3-Propylen, durch 1 oder 2 -OH substituiertes 1,2-Ethylen oder durch 1 oder 2 -OH substituiertes 1,3-Propylen, vor allem 1,2-Ethylen oder durch 1 oder 2 - OH substituiertes 1,2-Ethylen, dar.

Die Herstellung der erfindungsgemäßen Verbindungen kann in Analogie zu einem der in DE-A-2 536 335, GB-A-1 169 859 oder CH-A-408 033 beschriebenen Verfahren erfolgen. Zweckmäßig werden dazu ein Äquivalent eines Benztriazols der Formel II 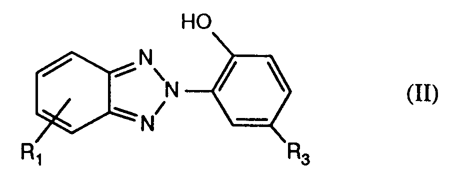 und ein Äquivalent eines Benztriazols der Formel II 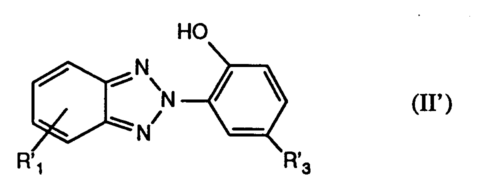 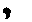 mit einem Äquivalent einer Verbindung der Formel III 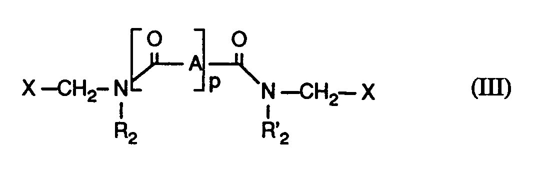 umgesetzt. A,R₁,R 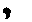 ₁,R₂,R 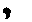 ₂,R₃,R 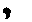 ₃ und p haben dabei die oben für Formel I angegebenen Bedeutungen; X steht für OH, Halogen oder Alkoxy. Die Temperatur der Umsetzung ist nicht kritisch und kann beispielsweise -10°C bis +150°C betragen; zweckmäßig findet die Umsetzung in einem geeigneten Reaktionsmedium (beispielsweise in einer Säure oder in einem organischen Lösemittel) in Gegenwart eines geeigneten Zusatzes (z. B. einer Säure, einer Alkaliverbindung, eines Dehydratisierungsmittels) statt, wobei Reaktionsmedium und Zusatz identisch sein können.

Vorzugsweise werden dazu Verbindungen der Formel III eingesetzt, worin X Hydroxy oder Chlor, insbesondere Hydroxy, bedeutet.

Zur Herstellung einer erfindungsgemäßen Verbindung der Formel I können beispielsweise Verbindungen der Formeln II und II 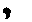 , die identisch oder verschieden sind, mit der Verbindung der Formel III in konzentrierter Schwefelsäure, z.B. mit 80-100 % Gehalt H₂SO₄, in einer Mischung aus Essigsäureanhydrid und Eisessig, in Polyphosphorsäure (ca. 100 bis 130 % Phosphorgehalt bezogen auf Orthophosphorsäure), oder in einem inerten Lösemittel wie beispielsweise Toluol oder Hexan unter Zusatz eines Dehydratisierungsmittels wie beispielsweise p-Toluolsulfonsäure oder Aluminiumchlorid, zweckmäßig unter Rühren, zur Reaktion gebracht werden. Bevorzugt wird Schwefelsäure verwendet und die Reaktion bei -5°C bis +40°C durchgeführt, wobei die Temperatur der Reaktionsmischung nach Zudosieren aller Komponenten bevorzugt im Bereich 0°C bis 30°C, vor allem 15°C bis 25°C (Raumtemperatur) gehalten wird. Die Aufarbeitung und Isolierung des Produktes kann auf bekannte Weise erfolgen, beispielsweise durch Verdünnen mit Wasser, zweckmäßig unter Kühlen (Eiswasser), Abfiltrieren des festen Produktes und anschließendem Waschen und Trocknen.

Beispiele für verwendbare Methylolverbindungen der Formel III sind u.a. N,N 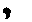 -Dimethylolharnstoff, N,N 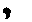 -Dimethylol-N,N 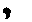 -dimethylharnstoff, N,N 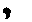 -Dimethylolharnstoff, 1,3-Dimethylol-tetrahydroimidazol-2-on (= 1,3-Dimethylol-ethylenharnstoff), 1,3-Dimethylol-4,5-dihydroxy-tetrahydroimidazol-2-on, N,N 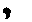 -Dimethylol-sebacinsäurediamid, N,N 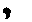 -Dimethylol-malonsäurediamid, N,N 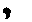 -Dimethylol-bemsteinsäurediamid.

Ebenfalls möglich ist die Herstellung von Verbindungen der Formel I durch Umsetzung von Verbindungen der oben wiedergegebenen Formeln II und II 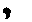 bei 0 bis 50°C in Schwefelsäure in Gegenwart von Formaldehyd mit einer Verbindung der Formel IV 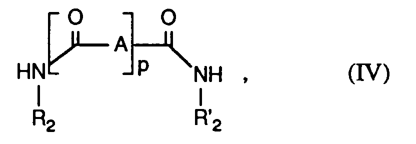 in Analogie zu einem in DE-A-2 536 335 beschriebenen Verfahren. Details der Reaktionsführung können dieser Publikation entnommen werden.

Die erfindungsgemäßen Verbindungen der Formel I eignen sich hervorragend zur Stabilisierung organischer Materialien, insbesondere organischer Polymere, gegen schädigende Einwirkung von Wärme, Sauerstoff und Licht, insbesondere UV-Strahlung. Beispiele für derartige organische Materialien sind in der folgenden Liste aufgeführt.
1. Polymere von Mono- und Diolefinen, beispielsweise Polypropylen, Polyisobutylen, Polybuten-1, Poly-4-methylpenten-1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE), verzweigtes Polyethylen niederer Dichte (VLDPE).
   Polyolefine, d.h. Polymere von Monoolefinen, wie sie beispielhaft im vorstehenden Absatz erwähnt sind, insbesondere Polyethylen und Polypropylen, können nach verschiedenen Verfahren hergestellt werden, insbesondere nach den folgenden Methoden:
   a) radikalisch (gewöhnlich bei hohem Druck und hoher Temperatur).
   b) mittels Katalysator, wobei der Katalysator gewöhnlich ein oder mehrere Metalle der Gruppe IVb, Vb, VIb oder VIII enthält. Diese Metalle besitzen gewöhnlich einen oder mehrere Liganden wie Oxide, Halogenide, Alkoholate, Ester, Ether, Amine, Alkyle, Alkenyle und/oder Aryle, die entweder π- oder σ-koordiniert sein können. Diese Metallkomplexe können frei oder auf Träger fixiert sein, wie beispielsweise auf aktiviertem Magnesiumchlorid, Titan(III)chlorid, Aluminiumoxid oder Siliziumoxid. Diese Katalysatoren können im Polymerisationsmedium löslich oder unlöslich sein. Die Katalysatoren können als solche in der Polymerisation aktiv sein, oder es können weitere Aktivatoren verwendet werden, wie beispielsweise Metallalkyle, Metallhydride, Metallalkylhalogenide, Metallalkyloxide oder Metallalkyloxane, wobei die Metalle Elemente der Gruppen Ia, IIa und/oder IIIa sind. Die Aktivatoren können beipielsweise mit weiteren Ester-, Ether-, Amin- oder Silylether-Gruppen modifiziert sein. Diese Katalysatorsysteme werden gewöhnlich als Phillips, Standard Oil Indiana, Ziegler (-Natta), TNZ (DuPont), Metallocen oder Single Site Katalysatoren (SSC) bezeichnet.
2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).
3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat- Copolymere, Ethylen-Vinylacetat-Copolymere und deren Copolymere mit Kohlenstoffmonoxid, oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere, LLDPE/Ethylen-Acrylsäure-Copolymere und alternierend oder statistisch aufgebaute Polyalkylen/Kohlenstoffmonoxid-Copolymere und deren Mischungen mit anderen Polymeren wie z.B. Polyamiden.
4. Kohlenwasserstoffharze (z.B. C₅-C₉) inklusive hydrierte Modifikationen davon (z.B. Klebrigmacherharze) und Mischungen von Polyalkylenen und Stärke.
5. Polystyrol, Poly-(p-methylstyrol), Poly-(α-methylstyrol).
6. Copolymere von Styrol oder α-Methylstyrol mit Dienen oder Acrylderivaten, wie z.B. Styrol-Butadien, Styrol-Acrylnitril, Styrol-Alkylmethacrylat, Styrol-Butadien-Alkylacrylat und -methacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.
7. Pfropfcopolymere von Styrol oder α-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 6) genannten Copolymeren, wie sie z.B. als sogenannte ABS-, MBS-, ASA- oder AES-Polymere bekannt sind.
8. Halogenhaltige Polymere, wie z.B. Polychloropren, Chlorkautschuk, chloriertes oder chlorsulfoniertes Polyethylen, Copolymere von Ethylen und chloriertem Ethylen, Epichlorhydrinhomo-und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.
9. Polymere, die sich von α,β-ungesättigten Säuren und deren Derivaten ableiten, wie Polyacrylate und Polymethacrylate, mit Butylacrylat schlagzäh modifizierte Polymethylmethacrylate, Polyacrylamide und Polyacrylnitrile.
10. Copolymere der unter 9) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-VinylhalogenidCopolymere oder Acrylnitril-Alkylmethacrylat-Butadien-Terpolymere.
11. Polymere, die sich von ungesättigten Alkoholen und Aminen bzw. deren Acylderivaten oder Acetalen ableiten, wie Polyvinylalkohol, Polyvinylacetat, -stearat, - benzoat, -maleat, Polyvinylbutyral, Polyallylphthalat, Polyallylmelamin; sowie deren Copolymere mit in Punkt 1 genannten Olefinen.
12. Homo- und Copolymere von cyclischen Ethern, wie Polyalkylenglykole, Polyethylenoxyd, Polypropylenoxyd oder deren Copolymere mit Bisglycidylethern.
13. Polyacetale, wie Polyoxymethylen, sowie solche Polyoxymethylene, die Comonomere, wie z.B. Ethylenoxid, enthalten; Polyacetale, die mit thermoplastischen Polyurethanen, Acrylaten oder MBS modifiziert sind.
14. Polyphenylenoxide und -sulfide und deren Mischungen mit Styrolpolymeren oder Polyamiden.
15. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.
16. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, 12/12, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid oder Poly-m-phenylen-isophthalamid. Block-Copolymere der vorstehend genanntenPolyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").
17. Polyharnstoffe, Polyimide, Polyamid-imide und Polybenzimidazole.
18. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.
19. Polycarbonate und Polyestercarbonate.
20. Polysulfone, Polyethersulfone und Polyetherketone.
21. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.
22. Trocknende und nicht-trocknende Alkydharze.
23. Ungesättigte Polyesterharze, die sich von Copolyestern gsättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.
24. Vernetzbare Acrylharze, die sich von substituierten Acrylsäureestern ableiten, wie z.B. von Epoxyacrylaten, Urethan-acrylaten oder Polyester-acrylaten.
25. Alkydharze, Polyesterharze und Acrylatharze, die mit Melaminharzen, Harnstoffharzen, Isocyanaten, Isocyanuraten, Polyisocyanaten oder Epoxidharzen vernetzt sind.
26. Vernetzte Epoxidharze, die sich von Polyepoxiden ableiten, z.B. von Bis-glycidylethern oder von cycloaliphatischen Diepoxiden.
27. Natürliche Polymere, wie Cellulose, Naturkautschuk, Gelatine, sowie deren polymerhomolog chemisch abgewandelte Derivate, wie Celluloseacetate, -propionate und - butyrate, bzw. die Celluloseether, wie Methylcellulose; sowie Kolophoniumharze und Derivate.
28. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.

Gegenstand der Erfindung ist daher auch eine Zusammensetzung enthaltend
A) ein gegen Schädigung durch Licht, Sauerstoff und/oder Hitze empfindliches organisches Material, insbesondere organisches Polymer, und
B) als Stabilisator eine Verbindung der Formel I.

Besonders vorteilhaft lassen sich die erfindungsgemäßen Verbindungen der Formel I einsetzen in Zusammensetzungen, die als Komponente A ein organisches Polymer, beispielsweise ein synthetisches organisches Polymer, enthalten. Von besonderer Bedeutung sind die Verbindungen der Formel I zur Stabilisierung von Zusammensetzungen, die ein thermoplastisches Polymer enthalten.

Die erfindungsgemäßen Zusammensetzungen können Bestandteil einer Überzugszusammensetzung, beispielsweise eines Lackes, oder einer Kunststoffzusammensetzung sein.

Bevorzugt sind solche Zusammensetzungen, worin Komponente A
i) ein thermoplastisches Polymer ist, ausgewählt aus organischen Polymeren, die Heteroatome, insbesondere Stickstoff und/oder Sauerstoff, in der Hauptkette enthalten, Styrol-Copolymeren, Styrol-Pfropfcolymeren und Polymethylmethacrylaten (PMMA); oder
ii) ein Lackbindemittel ist.

Thermoplastische Polymere, die Heteroatome in der Hauptkette enthalten, sind vor allem O, S und N enthaltende Polymere. Beispiele dafür finden sich in den Punkten 13 bis 20 der obenstehenden Liste. Bevorzugt sind darunter die Polycarbonate, Polyester, Polyamide, Polyacetale, Polyphenylenoxide und Polyphenylensulfide; insbesondere Polycarbonate, Polyester wie beispielsweise Polyethylenterephthalat (PET), und Polyamide (PA) wie beispielsweise PA 6 oder PA 6/6; vor allem aber die Polycarbonate.

Beispiele für Styrol-Copolymere und Styrol-Pfropfcolymere finden sich in den Punkten 6 und 7 der obenstehenden Liste.

Die Bindemittel für Lacke können mindestens eines der in obiger Liste aufgeführten Polymeren enthalten. Beispiele für Lacke mit speziellen Bindemitteln sind die folgenden:
1. Lacke auf Basis von kalt- oder heiß-vernetzbaren Alkyd-, Acrylat-, Polyester-, Epoxid-oder Melaminharzen oder Mischungen solcher Harze, gegebenenfalls mit Zusatz eines Härtungskatalysators;
2. Zweikomponenten-Polyurethanlacke auf Basis von hydroxylgruppenhaltigen Acrylat-, Polyester- oder Polyetherharzen und aliphatischen oder aromatischen Isocyanaten, Isocyanuraten oder Polyisocyanaten;
3. Einkomponenten-Polyurethanlacke auf Basis von blockierten Isocyanaten, Isocyanuraten oder Polyisocyanaten, die während des Einbrennens deblockiert werden;
4. Zweikomponentenlacke auf Basis von (Poly)ketiminen und aliphatischen oder aromatischen Isocyanaten, Isocyanuraten oder Polyisocyanaten;
5. Zweikomponentenlacke auf Basis von (Poly)ketiminen und einem ungesättigten Acrylatharz oder einem Polyacetoacetatharz oder einem Methacrylamidoglykolatmethylester;
6. Zweikomponentenlacke auf Basis von carboxyl- oder aminogruppenhaltigen Polyacrylaten und Polyepoxiden;
7. Zweikomponentenlacke auf Basis von anhydridgruppenhaltigen Acrylatharzen und einer Polyhydroxy- oder Polyaminokomponente;
8. Zweikomponentenlacke auf Basis von (Poly)oxazolinen und anhydridgruppenhaltigen Acrylatharzen oder ungesättigten Acrylatharzen oder aliphatischen oder aromatischen Isocyanaten, Isocyanuraten oder Polyisocyanaten;
9. Zweikomponentenlacke auf Basis von ungesättigten Polyacrylaten und Polymalonaten;
10. thermoplastische Polyacrylatlacke auf Basis von thermoplastischen Acrylatharzen oder fremdvernetzenden Acrylatharzen in Kombination mit veretherten Melaminharzen;
11. Lacksysteme auf Basis von siloxanmodifizierten Acrylatharzen;
12. Lacksysteme auf Basis von fluormodifizierten Acrylatharzen, und
13. Lacksysteme auf Basis von Allylglycidylethern.

Die Lacke können als Einschicht- oder Zweischichtlacke appliziert werden, wobei die erfindungsgemässen Stabilisatoren vorzugsweise der unpigmentierten obersten Schicht zugesetzt werden.

Die Lacke können auf die Substrate (Metall, Plastik, Holz etc.) nach den üblichen Verfahren aufgebracht werden, beispielsweise durch Streichen, Besprühen, Giessen, Tauchen oder Elektrophorese.

Eine bevorzugte Ausführungsform der vorliegenden Erfindung sind somit Anstrichstoffe bzw. Lacke (z.B. Automobillacke), die mindestens eine erfindungsgemässe Verbindung enthalten. Als Bindemittel kommen beispielsweise die vorstehend genannten in Betracht.

Die Erfindung betrifft auch ein Verfahren zum Stabilisieren von organischem Material, insbesondere organischen Polymeren, gegen Schädigung durch Licht, Sauerstoff und/oder Hitze, dadurch gekennzeichnet, daß man diesem als Stabilisator eine Verbindung der Formel I zusetzt, sowie die Verwendung von Verbindungen der Formel I zum Stabilisieren von organischem Material.

Die Menge des zu verwendenden Stabilisators richtet sich nach dem zu stabilisierenden organischen Material und der beabsichtigten Verwendung des stabilisierten Materials. Im allgemeinen enthält die erfindungsgemäße Zusammensetzung auf 100 Gew.-Teile der Komponente A 0,01 bis 15, besonders 0,1 bis 5 Gew.-Teile des Stabilisators (Komponente B).

Die Einarbeitung in die organischen Polymere kann beispielsweise durch Einmischen der erfindungsgemässen Verbindungen und gegebenenfalls weiterer Additive nach den in der Technik üblichen Methoden erfolgen. Die Einarbeitung kann vor oder während der Formgebung, beispielsweise durch Mischen der pulverförmigen Komponenten oder durch Zusatz des Stabilisators zur Schmelze oder Lösung des Polymeren, oder durch Aufbringen der gelösten oder dispergierten Verbindungen auf das Polymere, gegebenenfalls unter nachträglichem Verdunsten des Lösungsmittels erfolgen. Im Fall von Elastomeren können diese auch als Latices stabilisiert werden. Eine weitere Möglichkeit der Einarbeitung der erfindungsgemässen Verbindungen in Polymere besteht in deren Zugabe vor oder während der Polymerisation der entsprechenden Monomeren bzw. vor der Vernetzung.

Die erfindungsgemässen Verbindungen oder Mischungen davon können auch in Form eines Masterbatches, der diese Verbindungen beispielsweise in einer Konzentration von 2,5 bis 25 Gew.% enthält, den zu stabilisierenden Kunststoffen zugesetzt werden.

Zweckmässig kann die Einarbeitung der erfindungsgemässen Verbindungen nach folgenden Methoden erfolgen:
- als Emulsion oder Dispersion (z.B. zu Latices oder Emulsionspolymeren)
- als Trockenmischung während des Vermischens von Zusatzkomponenten oder Polymermischungen
- durch direktes Zugeben in die Verarbeitungsapparatur (z.B. Extruder, Innenmischer usw.)
- als Lösung oder Schmelze.

Die so erhaltenen stabilisierten Polymerzusammensetzungen können nach den üblichen Methoden, wie z.B. durch Heißpressen, Spinnen, Extrudieren oder Spritzgießen, in geformte Gegenstände überführt werden, wie z.B. in Fasern, Folien, Bändchen, Platten, Stegplatten, Gefäße, Rohre und sonstige Profile.

Die Erfindung betrifft daher weiterhin die Verwendung der erfindungsgemäßen Polymerzusammensetzung zur Herstellung eines geformten Gegenstandes.

Von Interesse ist auch die Verwendung in Mehrschichtsystemen. Hierbei wird eine erfindungsgemäße Polymerenzusammensetzung mit einem relativ hohen Gehalt an Stabilisator der Formel Ib, beispielsweise 5 - 15 Gew.-%, in dünner Schicht (10-100 µm) auf einen geformten Gegenstand aus einem Polymer, das wenig oder keinen Stabilisator der Formel Ib enthält, aufgebracht. Das Aufbringen kann zugleich mit der Formgebung des Grundkörpers geschehen, z.B. durch sogenannte Coextrusion. Das Aufbringen kann aber auch auf den fertig geformten Grundkörper geschehen, z.B. durch Lamination mit einem Film oder durch Beschichtung mit einer Lösung. Die äußere Schicht bzw. die äußeren Schichten des fertigen Gegenstandes haben die Funktion eines UV-Filters, der das Innere des Gegenstandes gegen UV-Licht schützt. Die äußere Schicht enthält vorzugsweise 5-15 Gew.%, insbesondere 5-10 Gew.%, mindestens eines Stabilisators der Formel Ib.

Die Verwendung der erfindungsgemäßen Polymerzusammensetzung zur Herstellung von Mehrschichtsystemen, wobei die äußere(n) Schicht(en) in einer Dicke von 10-100 µm aus einer erfindungsgemäßen Polymerzusammensetzung besteht, während die innere Schicht wenig oder keinen Stabilisator der Formel Ib enthält, stellt daher einen weiteren Gegenstand der Erfindung dar.

Die so stabilisierten Polymere zeichnen sich aus durch hohe Witterungsbeständigkeit, vor allem durch hohe Beständigkeit gegen UV-Licht. Sie behalten dadurch auch im Außengebrauch lange Zeit ihre mechanischen Eigenschaften sowie ihre Farbe und ihren Glanz.

Der Stabilisator (Komponente B) kann auch ein Gemisch sein von zwei oder mehr erfindungsgemäßen Verbindungen. Die erfindungsgemäßen Zusammensetzungen, stabilisierten Überzugsmittel oder organischen Polymere, können außer dem Stabilisator der Formel I noch andere Stabilisatoren und/oder sonstige Zusätze enthalten, wie z.B. Antioxidantien, weitere Lichtschutzmittel, Metalldesaktivatoren, Phosphite oder Phosphonite. Beispiele hierfür sind die folgenden Stabilisatoren:
1. Antioxidantien
   1.1. Alkylierte Monophenole, z.B. 2,6-Di-tert-butyl-4-methylphenol, 2-butyl-4,6-dimethylphenol, 2,6-Di-tert-butyl-4-ethylphenol, 2,6-Di-tert-butyl-4-n-butylphenol, 2,6-Di-tert-butyl-4-iso-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert-butyl-4-methoxymethylphenol, 2,6-Di-nonyl-4-methylphenol, 2,4-Dimethyl-6-(1'-methyl-undec-1'-yl)-phenol, 2,4-Dimethyl-6-(1'-methyl-heptadec-1'-yl)-phenol, 2,4-Dimethyl-6-(1'-methyl-tridec-1'-yl)-phenol und Mischungen davon.
   1.2. Alkylthiomethylphenole, z.B. 2,4-Di-octylthiomethyl-6-tert-butylphenol, 2,4-Di-octylthiomethyl-6-methylphenol, 2,4-Di-octylthiomethyl-6-ethylphenol, 2,6-Di-dodecylthiomethyl-4-nonylphenol.
   1.3. Hydrochinone und alkylierte Hydrochinone, z.B. 2,6-Di-tert-butyl-4-methoxyphenol, 2,5-Di-tert-butyl-hydrochinon, 2,5-Di-tert-amyl-hydrochinon, 2,6-Diphenyl-4-octadecyloxyphenol, 2,6-Di-tert-butyl-hydrochinon, 2,5-Di-tert-butyl-4-hydroxyanisol, 3,5-Di-tert-butyl-4-hydroxyanisol, 3,5-Di-tert-butyl-4-hydroxyphenyl-stearat, Bis-(3,5-di-tert-butyl-4-hydroxyphenyl)adipat.
   1.4. Tocopherole, z.B. α-Tocopherol, β-Tocopherol, γ-Tocopherol, δ-Tocopherol und Mischungen davon (Vitamin E).
   1.5. Hydroxylierte Thiodiphenylether, z.B. 2,2 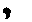 -Thio-bis-(6-tert-butyl-4-methylphenol), 2,2 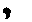 -Thio-bis-(4-octylphenol), 4,4 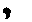 -Thio-bis-(6-tert-butyl-3-methylphenol), 4,4 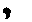 -Thio-bis-(6-tert-butyl-2-methylphenol), 4,4'-Thio-bis-(3,6-di-sec.-amylphenol), 4,4'-Bis-(2,6-dimethyl-4-hydroxyphenyl)-disulfid.
   1.6. Alkyliden-Bisphenole, z.B. 2,2 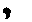 -Methylen-bis-(6-tert-butyl-4-methylphenol), 2,2 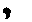 -Methylen-bis-(6-tert-butyl-4-ethylphenol), 2,2 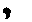 -Methylen-bis-[4-methyl-6-(α-methylcyclohexyl)-phenol], 2,2 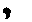 -Methylen-bis-(4-methyl-6-cyclohexylphenol), 2,2 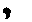 -Methylen-bis-(6-nonyl-4-methylphenol), 2,2 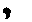 -Methylen-bis-(4,6-di-tert-butylphenol), 2,2 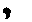 -Ethyliden-bis-(4,6-di-tert-butylphenol), 2,2 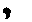 -Ethyliden-bis-(6-tert-butyl-4-isobutylphenol), 2,2 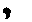 -Methylen-bis-[6-(α-methylbenzyl)-4-nonylphenol], 2,2 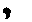 -Methylen-bis-[6-(α,α-dimethylbenzyl)-4-nonylphenol], 4,4 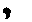 -Methylen-bis-(2,6-di-tert-butylphenol), 4,4 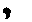 -Methylen-bis-(6-tert-butyl-2-methylphenol), 1,1-Bis-(5-tert-butyl-4-hydroxy-2-methylphenyl)-butan, 2,6-Bis-(3-tert-butyl-5-methyl-2-hydroxybenzyl)-4-methylphenol, 1,1,3-Tris-(5-tert-butyl-4-hydroxy-2-methylphenyl)-butan, 1,1-Bis-(5-tert-butyl-4-hydroxy-2-methylphenyl)-3-n-dodecylmercaptobutan, Ethylenglycol-bis-[3,3-bis-(3 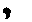 -tert-butyl-4 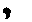 -hydroxyphenyl)-butyrat], Bis-(3-tert-butyl-4-hydroxy-5-methyl-phenyl)-dicyclopentadien, Bis-[2-(3 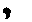 -tert-butyl-2 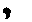 -hydroxy-5 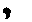 -methyl-benzyl)-6-tert-butyl-4-methyl-phenyl]-terephthalat, 1,1-Bis-(3,5-dimethyl-2-hydroxyphenyl)-butan, 2,2-Bis-(3,5-di-tert-butyl-4-hydroxyphenyl)-propan, 2,2-Bis-(5-tert-butyl-4-hydroxy-2-methylphenyl)-4-n-dodecylmercaptobutan, 1,1,5,5-Tetra-(5-tert-butyl-4-hydroxy-2-methylphenyl)-pentan.
   1.7. O-, N- und S-Benzylverbindungen, z.B. 3,5,3',5'-Tetra-tert-butyl-4,4'-dihydroxydibenzylether, Octadecyl-4-hydroxy-3,5-dimethylbenzyl-mercaptoacetat, Tris-(3,5-di-tert-butyl-4-hydroxybenzyl)-amin, Bis-(4-tert-butyl-3-hydroxy-2,6-dimethylbenzyl)-dithioterephthalat, Bis-(3,5-di-tert-butyl-4-hydroxybenzyl)-sulfid, Isooctyl-3,5-di-tert-butyl-4-hydroxybenzyl-mercaptoacetat.
   1.8. Hydroxybenzylierte Malonate, z.B. Dioctadecyl-2,2-bis-(3,5-di-tert-butyl-2-hydroxybenzyl)-malonat, Di-octadecyl-2-(3-tert-butyl-4-hydroxy-5-methylbenzyl)-malonat, Di-dodecylmercaptoethyl-2,2-bis-(3,5-di-tert-butyl-4-hydroxybenzyl)-malonat, Di-[4-(1,1,3,3-tetramethylbutyl)-phenyl]-2,2-bis-(3,5-di-tert-butyl-4-hydroxybenzyl)-malonat.
   1.9. Hydroxybenzyl-Aromaten, z.B. 1,3,5-Tris-(3,5-di-tert-butyl-4-hydroxybenzyl)-2,4,6-trimethylbenzol, 1,4-Bis-(3,5-di-tert-butyl-4-hydroxybenzyl)-2,3,5,6-tetramethylbenzol, 2,4,6-Tris-(3,5-di-tert-butyl-4-hydroxybenzyl)-phenol.
   1.10. Triazinverbindungen, z.B. 2,4-Bis-octylmercapto-6-(3,5-di-tert-butyl-4-hydroxyanilino)-1,3, 5-triazin, 2-Octylmercapto-4,6-bis-(3,5-di-tert-butyl-4-hydroxyanilino)-1,3,5-triazin, 2-Octylmercapto-4,6-bis-(3,5-di-tert-butyl-4-hydroxyphenoxy)-1,3,5-triazin, 2,4,6-Tris-(3,5-di-tert-butyl-4-hydroxyphenoxy)-1,2,3-triazin, 1,3,5-Tris-(3,5-di-tert-butyl-4-hydroxybenzyl)-isocyanurat, 1,3,5-Tris-(4-tert-butyl-3-hydroxy-2,6-dimethylbenzyl)-isocyanurat, 2,4,6-Tris-(3,5-di-tert-butyl-4-hydroxyphenylethyl)-1,3,5-triazin, 1,3,5-Tris-(3,5-di-tert-butyl-4-hydroxyphenylpropionyl)-hexahydro-1,3,5-triazin, 1,3,5-Tris-(3,5-dicyclohexyl-4-hydroxybenzyl)-isocyanurat.
   1.11. Benzylphosphonate, z.B. Dimethyl-2,5-di-tert-butyl-4-hydroxybenzylphosphonat, Diethyl-3,5-di-tert-butyl-4-hydroxybenzylphosphonat, Dioctadecyl-3,5-di-tert-butyl-4-hydroxybenzylphosphonat, Dioctadecyl-5-tert-butyl-4-hydroxy-3-methylbenzylphosphonat, Ca-Salz des 3,5-Di-tert-butyl-4-hydroxybenzyl-phosphonsäure-monoethylesters.
   1.12. Acylaminophenole, z.B. 4-Hydroxy-laurinsäureanilid, 4-Hydroxystearinsäureanilid, N-(3,5-di-tert-butyl-4-hydroxyphenyl)-carbaminsäureoctylester.
   1.13. Ester der β-(3,5-Di-tert-butyl-4-hydroxyphenyl)-propionsäure mit ein- oder mehr wertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxyethyl)-isocyanurat, N,N 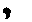 -Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
   1.14. Ester der β-(5-tert-Butyl-4-hydroxy-3-methylphenyl)-propionsäure mit ein- oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N 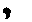 -Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
   1.15. Ester der β-(3,5-Dicyclohexyl-4-hydroxyphenyl)-propionsäure mit ein-oder mehr wertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N 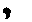 -Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
   1.16. Ester der 3,5-Di-tert-butyl-4-hydroxyphenylessigsäure mit ein-oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N 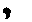 -Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo[2.2.2]-octan.
   1.17. Amide der β-(3,5-Di-tert-butyl-4-hydroxyphenyl)-propionsäure, wie z.B. N,N 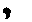 -Bis-(3,5-di-tert-butyl-4-hydroxyphenylpropionyl)-hexamethylendiamin, N,N 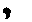 -Bis-(3,5-di-tert-butyl-4-hydroxyphenylpropionyl)-trimethylendiamin, N,N 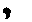 -Bis-(3,5-di-tert-butyl-4-hydroxyphenylpropionyl)-hydrazin.
2. UV-Absorber und Lichtschutzmittel
   2.1. 2-(2 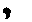 -Hydroxyphenyl)-benzotriazole, wie z.B. 2-(2 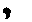 -Hydroxy-5 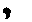 -methylphenyl)-benzotriazol, 2-(3 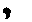 ,5 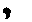 -Di-tert-butyl-2 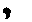 -hydroxyphenyl)-benzotriazol, 2-(5 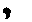 -tert-Butyl-2 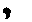 -hydroxyphenyl)-benzotriazol, 2-(2 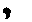 -Hydroxy-5 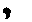 -(1,1,3,3-tetramethylbutyl)phenyl)-benzotriazol, 2-(3 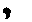 ,5 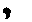 -Di-tert-butyl-2 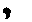 -hydroxyphenyl)-5-chlor-benzotriazol, 2-(3 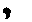 -tert-Butyl-2 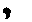 -hydroxy-5 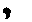 -methylphenyl)-5-chlor-benzotriazol, 2-(3 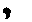 -sec-Butyl-5 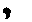 -tert-butyl-2 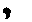 -hydroxyphenyl)-benzotriazol, 2-(2 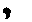 -Hydroxy-4 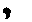 -octoxyphenyl)-benzotriazol, 2-(3 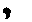 ,5 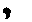 -Di-tert-amyl-2 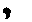 -hydroxyphenyl)-benzotriazol, 2-(3 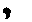 ,5 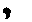 -Bis-(α,α-dimethylbenzyl)-2 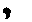 -hydroxyphenyl)-benzotriazol, Mischung aus 2-(3'-tert-Butyl-2 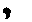 -hydroxy-5'-(2-octyloxycarbonylethyl)-phenyl)-5-chlor-benzotriazol, 2-(3'-tert-Butyl-5'-[2-(2-ethylhexyloxy)-carbonylethyl]-2 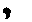 -hydroxyphenyl)-5-chlor-benzotriazol, 2-(3'-tert-Butyl-2 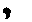 -hydroxy-5'-(2-methoxycarbonylethyl)phenyl)-5-chlor-benzotriazol, 2-(3'-tert-Butyl-2 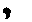 -hydroxy-5'-(2-methoxycarbonylethyl)phenyl)-benzotriazol, 2-(3'-tert-Butyl-2 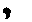 -hydroxy-5'-(2-octyloxycarbonylethyl)phenyl)-benzotriazol, 2-(3'-tert-Butyl-5'-[2-(2-ethylhexyloxy)carbonylethyl]-2 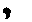 -hydroxyphenyl)-benzotriazol, 2-(3'-Dodecyl-2 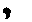 -hydroxy-5'-methylphenyl)-benzotriazol, und 2-(3'-tert-Butyl-2'-hydroxy-5'-(2-isooctyloxycarbonylethyl)phenyl-benzotriazol, 2,2'-Methylen-bis[4-(1,1,3,3-tetramethylbutyl)-6-benzotriazol-2-yl-phenol]; Umesterungsprodukt von 2-[3'-tert-Butyl-5'-(2-methoxycarbonylethyl)-2'-hydroxy-phenyl]-benzotriazol mit Polyethylenglycol 300; [R-CH₂CH₂-COO(CH₂)₃⁆₂ mit R = 3'-tert-Butyl-4'-hydroxy-5'-2H-benzotriazol-2-yl-phenyl.
   2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2 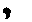 ,4 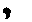 -Trihydroxy-, 2 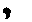 -Hydroxy-4,4 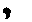 -dimethoxy-Derivat.
   2.3. Ester von gegebenenfalls substituierten Benzoesäuren, wie z.B. 4-tert-Butyl-phenyl-salicylat, Phenylsalicylat, Octylphenyl-salicylat, Dibenzoylresorcin, Bis-(4-tert-butylbenzoyl)-resorcin, Benzoylresorcin, 3,5-Di-tert-butyl-4-hydroxybenzoesäure-2,4-di-tert-butylphenylester, 3,5-Di-tert-butyl-4-hydroxybenzoesäurehexadecylester, 3,5-Di-tert-butyl-4-hydroxybenzoesäure-octadecylester, 3,5-Di-tert-butyl-4-hydroxybenzoesäure-2-methyl-4,6-di-tert-butylphenylester.
   2.4. Acrylate, wie z.B. α-Cyan-β,β-diphenylacrylsäure-ethylester bzw. - isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.
   2.5. Nickelverbindungen, wie z.B. Nickelkomplexe des 2,2 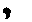 -Thio-bis-[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert-butylbenzylphosphonsäure-monoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.
   2.6. Sterisch gehinderte Amine, wie z.B. Bis-(2,2,6,6-tetramethyl-piperidyl)-sebacat, Bis-(2,2,6,6-tetramelhyl-piperidyl)-succinat, Bis-(1,2,2,6,6-pentamethylpiperidyl)-sebacat, n-Butyl-3,5-di-tert-butyl-4-hydroxybenzyl-malonsäure-bis(1,2, 2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N 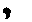 -Bis-(2,2,6,6-Tetramethyl-4-piperidyl)-hexamethylendiamin und 4-tert-Octylamino-2,6-dichlor-1,3,5-s-triazin, Tris-(2,2,6,6-tetramethyl-4-piperidyl)-nitrilotriacetat, Tetrakis-(2,2,6,6-tetramethyl-4-piperidyl)-1,2,3,4-butantetraoat, 1,1 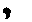 -(1,2-Ethandiyl)-bis-(3,3,5,5-tetramethyl-piperazinon), 4-Benzoyl-2,2,6,6-tetramethylpiperidin, 4-Stearyloxy-2,2,6,6-tetramethylpiperidin, Bis-(1,2,2,6,6-pentamethylpiperidyl)-2-n-butyl-2-(2-hydroxy-3,5-di-tert-butylbenzyl)-malonat, 3-n-Octyl-7,7,9,9-tetramethyl-1,3,8-triazaspiro[4.5]decan-2,4-dion, Bis-(1-octyloxy-2,2,6,6-tetramethylpiperidyl)-sebacat, Bis-(1-octyloxy-2,2,6,6-tetramethylpiperidyl)-succinat, Kondensationsprodukt aus N,N'-Bis-(2,2,6,6-tetramethyl-4-piperidyl)-hexamethylendiamin und 4-Morpholino-2,6-dichlor-1,3,5-triazin, Kondensationsprodukt aus 2-Chlor-4,6-di-(4-n-butylamino-2,2,6,6-tetramethylpiperidyl)-1,3,5-triazin und 1,2-Bis-(3-aminopropylamino)äthan, Kondensationsprodukt aus 2-Chlor-4,6-di-(4-n-butylamino-1,2,2,6,6-pentamethylpiperidyl)-1,3,5-triazin und 1,2-Bis-(3-aminopropylamino)-äthan, 8-Acetyl-3-dodecyl-7,7,9,9-tetramethyl-1,3,8-triazaspiro[4.5]decan-2,4-dion, 3-Dodecyl-1-(2,2,6,6-tetramethyl-4-piperidyl)pyrrolidin-2,5-dion, 3-Dodecyl-1-(1,2,2,6,6-pentamethyl-4-piperidyl)-pyrrolidin-2,5-dion.
   2.7. Oxalsäurediamide, wie z.B. 4,4 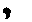 -Di-octyloxy-oxanilid, 2,2 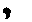 -Diethoxy-oxanilid, 2,2 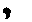 -Di-octyloxy-5,5 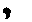 -di-tert-butyl-oxanilid, 2,2 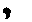 -Di-dodecyloxy-5,5 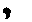 di-tert-butyl-oxanilid, 2-Ethoxy-2 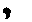 -ethyl-oxanilid, N,N 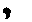 -Bis-(3-dimethylaminopropyl)-oxalamid, 2-Ethoxy-5-tert-butyl-2 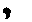 -ethyloxanilid und dessen Gemisch mit 2-Ethoxy-2 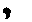 -ethyl-5,4 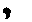 -di-tert-butyl-oxanilid, Gemische von o- und p-Methoxy- sowie von o- und p-Ethoxy-di-substituierten Oxaniliden.
   2.8. 2-(2-Hydroxyphenyl)-1,3,5-triazine, wie z.B. 2,4,6-Tris(2-hydroxy-4-octyloxyphenyl)-1,3,5-triazin, 2-(2-Hydroxy-4-octyloxyphenyl)-4,6-bis-(2,4-dimethylphenyl)-1,3,5-triazin, 2-(2,4-Dihydroxyphenyl)-4,6-bis(2,4-dimethylphenyl)-1,3,5-triazin, 2,4-Bis-(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin, 2-[2-hydroxy-4-(2-hydroxy-3-butyloxy-propyloxy)phenyl]-4,6-bis(2,4-dimethylphenyl)-1,3,5-triazin, 2-[2-hydroxy-4-(2-hydroxy-3-octyloxy-propyloxy)phenyl]-4,6-bis(2,4-dimethylphenyl)-1,3,5-triazin.
3. Metalldesaktivatoren, wie z.B. N,N 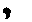 -Diphenyloxalsäurediamid, N-Salicylal-N 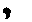 -salicyloylhydrazin, N,N 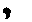 -Bis-(salicyloyl)-hydrazin, N,N 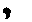 -Bis-(3,5-di-tert-butyl-4-hydroxyphenylpropionyl)-hydrazin, 3-Salicyloylamino-1,2,4-triazol, Bis-(benzyliden)-oxalsäuredihydrazid, Oxanilid, Isophthalsäure-dihydrazid, Sebacinsäure-bis-phenylhydrazid, N,N'-Diacetyl-adipinsäure-dihydrazid, N,N'-Bis-salicyloyl-oxalsäure-dihydrazid, N,N'-Bis-salicyloyl-thiopropionsäure-dihydrazid.
4. Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert-butylphenyl)-phosphit, Diisodecylpentaerythrit-diphosphit, Bis-(2,4-di-tert-butylphenyl)-pentaerythritdiphosphit, Bis-(2,6-di-tert-butyl-4-methylphenyl)-pentaerythritdiphosphit, Bis-isodecyloxy-pentaerythrit-diphosphit, Bis-(2,4-di-tert-butyl-6-methylphenyl)-pentaerythritdiphosphit, Bis-(2,4,6-tri-tert-butylphenyl)-pentaerythritdiphosphit, Tristearyl-sorbit-triphosphit, Tetrakis-(2,4-di-tert-butylphenyl)-4,4 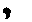 -biphenylen-diphosphonit, 6-Isooctyloxy-2,4,8,10-tetra-tert-butyl-12H-dibenz[d,g]-1,3,2-dioxaphosphocin, 6-Fluor-2,4,8,10-tetra-tert-butyl-12-methyl-dibenz[d,g]-1,3,2-dioxaphosphocin, Bis-(2,4-di-tert-butyl-6-methylphenyl)-methylphosphit, Bis-(2,4-di-tert-butyl-6-methylphenyl)-ethylphosphit.
5. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.
6. Polyamidstabilisatoren, wie z.B. Kupfersalze in Kombination mit Jodiden und/oder Phosphorverbindungen und Salze des zweiwertigen Mangans.
7. Basische Co-Stabilisatoren, wie z.B. Melamin, Polyvinylpyrrolidon, Dicyandiamid, Tri-allylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Behenat, Mg-Stearat, Na-Ricinoleat, K-Palmitat, Antimonbrenzcatechinat oder Zinnbrenzcatechinat.
8. Nukleierungsmittel, wie z.B. 4-tert-Butylbenzoesäure, Adipinsäure, Diphenylessigsäure.
9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Ruß, Graphit.
10. Sonstige Zusätze, wie z.B. Weichmacher, Gleitmittel, Emulgatoren, Pigmente, Optische Aufheller, Flammschutzmittel, Antistatika, Treibmittel.
11. Benzofuranone bzw. Indolinone, wie z.B. in US-A-4 325 863, US-A-4 338 244, US-A-5 175 312, US-A-5 216 052, US-A-5 252 643, DE-A-4 316 611, DE-A-4 316 622, DE-A-4 316 876, EP-A-0 589 839 oder EP-A-0 591 102 beschrieben, oder 3-[4-(2-Acetoxyethoxy)phenyl]-5,7-di-tert-butyl-benzofuran-2-on, 5,7-Di-tert-butyl-3-[4-(2-stearoyloxyethoxy)phenyl]-benzofuran-2-on, 3,3 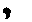 -Bis-[5,7-di-tert-butyl-3-(4-[2-hydroxyethoxy]-phenyl)-benzofuran-2-on], 5,7-Di-tert-butyl-3-(4-ethoxyphenyl)benzofuran-2-on, 3-(4-Acetoxy-3,5-dimethylphenyl)-5,7-di-tert-butyl-benzofuran-2-on, 3-(3,5-Dimethyl-4-pivaloyloxy-phenyl)-5,7-di-tert-butyl-benzofuran-2-on.

Die Art und Menge der zugesetzten weiteren Stabilisatoren wird von der Art des zu stabilisierenden Substrates und dessen Verwendungszweck bestimmt; häufig werden 0,1 bis 5 Gew.-% bezogen auf das zu stabilisierende Polymer verwendet.

Die folgenden Beispiele beschreiben die erfindungsgemäßen Überzugsmittel weiter, ohne die Erfindung auf die Beispiele zu beschränken. Darin bedeuten Teile Gewichtsteile und Angaben in % Gewichts-%.

### Beispiel 1

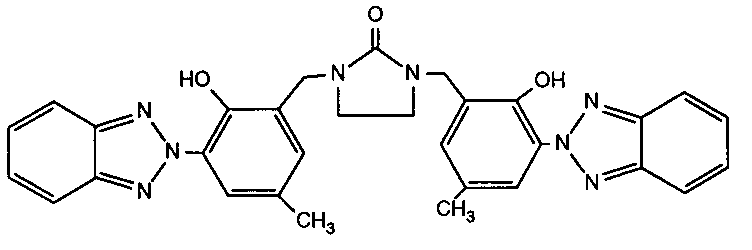

45g (0,2Mol) 2-(2 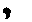 -Hydroxy-5 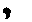 -methyl)-benztriazol werden bei 0° bis -5°C in 200ml konz. Schwefelsäure vorgelegt. Dann werden 29,2g (0,1Mol) 1,3-Dimethylol-ethylen-harnstoff (50% Lösung in Wasser) innerhalb von 20 Minuten bei -5° bis 0°C zugetropft.

Anschliessend wird noch 2 Stunden bei +20°C gerührt, auf 11 Eiswasser gegossen, abfiltriert und mit Wasser neutral gewaschen. Das Nutschgut wird mit 600ml Dimethylacetamid verrührt, auf 100°C erwärmt, abgekühlt und dann abfiltriert. Nach dem Waschen mit Dimethylacetamid und Wasser und anschliessendem Trocknen bei 100°C im Vakuumtrockenschrank erhält man 42g eines weissen Pulvers vom Schmelzpunkt 283,5°C, entsprechend einer Ausbeute von 74,9%.

### Beispiel 2

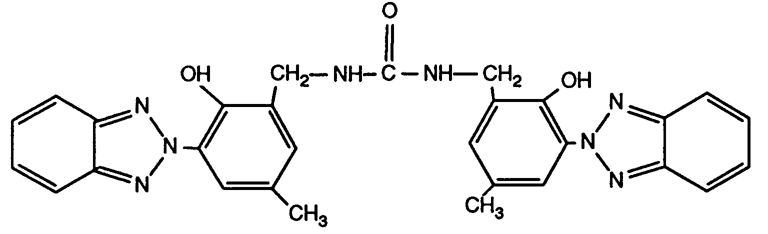

Man geht vor wie in Beispiel 1 beschrieben, aber unter Verwendung von 12g (0,1 Mol) Bis-Methylolharnstoff anstelle von 1,3-Dimethylolethylenharnstoff. Man erhält 52g eines weissen Produktes vom Schmelzpunkt 310,4°C, entsprechend einer Ausbeute von 97,3%.

### Beispiel 3

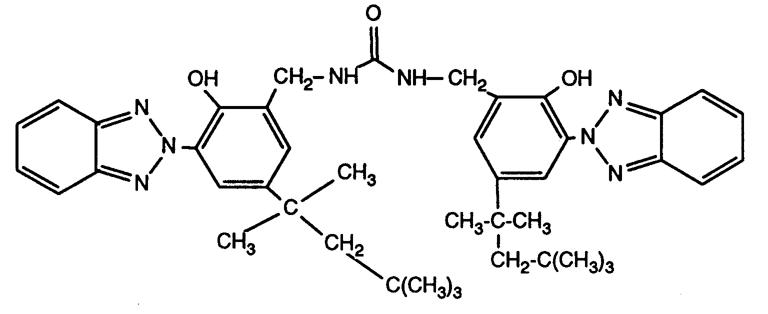

Es wird wie in Beispiel 2 beschrieben vorgegangen, jedoch unter Verwendung von 64,6g (0,2 Mol) 2-(2 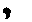 -Hydroxy-5 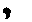 -tert.-oktyl)-benztriazol anstelle von 45g (0,2 Mol) 2-(2 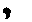 -Hydroxy-5 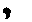 -methyl)-benztriazol. Nach der Aufarbeitung erhält man 21,5g eines weissen Produktes vom Schmelzpunkt 190°C.

### Beispiele 4-6

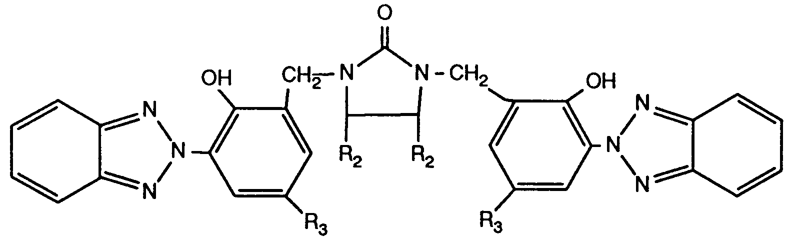

Nach der in Beispiel 1 beschriebenen Methode wurden die Verbindungen Nr. 4-6 der oben dargestellten allgemeinen Formel hergestellt. Struktur und physikalische Daten für diese Verbindungen sind nachfolgender Tabelle zu entnehmen.

**Tabelle**

| Verbindungen der Beispiele Nr. 4-6 | | | |
|---|---|---|---|
| Beispiel Nr. | R₃ | R₂ | Schmelzpunkt |
| 4 | tert.-Oktyl | H | 174,6°C |
| 5 | tert.-Oktyl | OH | 215°C |
| 6 | Methyl | OH | 338°C |

### Anwendungsbeispiele

### Beispiel 7: Stabilisierung von Polycarbonat (PC)

In 50 g Methylenchlorid werden 10 g Polycarbonat-Pulver (Lexan® 115) unter Rühren bei Raumtemperatur gelöst, was mehrere Stunden benötigt. Dazu kommen 0,2 g UV-Absorber entsprechend Beispiel 4 oder Beispiel 5, entsprechend 2 % Zusatzkonzentration. Zu Vergleichszwecken wird eine weitere Lösung ohne UV-Absorber hergestellt. Aus diesen Lösungen werden Filme von 20 µm Dicke gegossen.

Die Filme werden in einem Atlas Weatherometer CI 65 bei einer Schwarztafeltemperatur von 63°C und einer relativen Feuchte von 60 % belichtet. Vor Beginn der Bewitterung und anschließend in regelmäßigen Intervallen wird die Verfärbung der Proben durch Messung des Yellowness Index (YI, Methode ASTM D 1925) überprüft. Weiterhin werden die Proben auf Versprödung untersucht. Die Ergebnisse sind in Tabelle 1 zusammengestellt; YI(0) bezeichnet die Anfangsfarbe (= Yellowness-Index vor Beginn der Bewitterung), Versprödung der Probe ist mit dem Zeichen * markiert.

**Tabelle 1**

| Yellowness Index YI und Versprödung vor Beginn und während der Bewitterung; erfindungsgemäße Proben enthaltend 2 % UV-Absorber | | | | | | | | | |
|---|---|---|---|---|---|---|---|---|---|
| UV-Absorber | Bewitterungsdauer/h | | | | | | | | |
| | 0 | 552 | 1046 | 1548 | 2030 | 3000 | 3501 | 4502 | 5507 |
| keiner | 0,1 | 3,6 | 12,2* | | | | | | |
| Beispiel 4 | 0,2 | 1,6 | 3,0 | 9,2 | 9,3 | 10,8 | 12,1 | 12,2 | 12,9* |
| Beispiel 5 | 0,5 | | | | | | | | |

| | | | | | | | | | |
|---|---|---|---|---|---|---|---|---|---|
| * Versprödung der Probe | | | | | | | | | |

Die in Tabelle 1 wiedergegebenen Daten zeigen, daß ein Beimischen der erfindungsgemäßen Verbindungen zu praktisch keinen Verfärbungen des Polycarbonates führt. Im Bewitterungstest zeigen die erfindungsgemäßen Verbindungen eine hervorragende Wilksamkeit.

### Beispiel 8: Stabilisierung von Polymethylmethacrylat (PMMA)

15 g Polymethylmethacrylat und 60 mg bzw. 300 mg des erfindungsgemäßen Stabilisators (entsprechend 0,4% bzw. 2 % Stabilisator) werden in 85g Methylenchlorid bei Raumtemperatur gelöst Aus dieser Lösung werden auf Glasplatten Filme gezogen, die nach Verdunsten des Lösungsmittels und Trocknung im Vakuum eine Dicke von 30 µm haben.

Die Filme werden von den Glasplatten abgezogen und in Kartonrahmen (6 x 3 cm) gespannt. Diese Proben werden in einem UV-Belichtungsgerät mit 5 Leuchtstofflampen TL/09 und 5 Lampen TL/12, die 20 cm über den Proben montiert sind, 3 Monate bestrahlt. In regelmässigen Abständen wird bei der Wellenlänge der maximalen Extinktion die UV-Absorption gemessen. Weiterhin wird die Verfärbung der Proben durch Messung des Yellowness Index (YI, Methode ASTM D 1925) überprüft.

Die erfindungsgemäß stabilisierten Proben weisen eine hervorragende Lichtbeständigkeit auf.

## Patentansprüche

1. Verbindung der Formel I 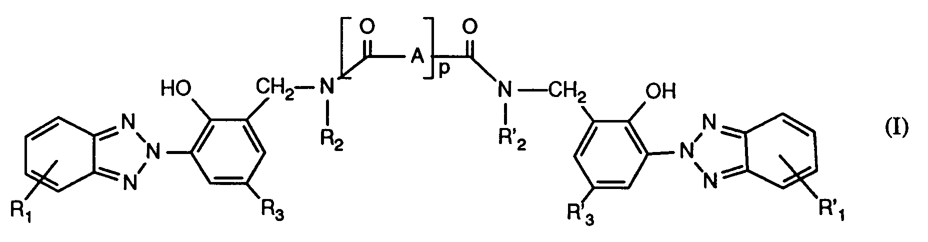
worin p 0 oder 1 ist;
A C₁-C₁₂-Alkylen darstellt;
R₁ und R 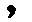 ₁ unabhängig voneinander Wasserstoff, Halogen, C₁-C₁₈-Alkyl, C₁-C₁₈-Alkoxy oder -CN bedeuten;
R₂ und R 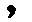 ₂ unabhängig voneinander Wasserstoff oder C₁-C₁₈-Alkyl oder gemeinsam C₂-C₁₂-Alkylen oder C₂-C₁₂-Hydroxyalkylen darstellen; und
R₃ und R 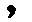 ₃ unabhängig voneinander Wasserstoff, Halogen, C₁-C₁₈-Alkyl oder C₁-C₁₈-Alkoxy bedeuten.

2. Verbindung der Formel I gemäß Anspruch 1, worin p 0 ist.

3. Verbindung der Formel I gemäß Anspruch 1, worin
R₁ und R 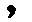 ₁ unabhängig voneinander Wasserstoff, Halogen, C₁-C₁₂-Alkyl, C₁-C₁₂-Alkoxy oder -CN bedeuten;
R₂ und R 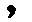 ₂ unabhängig voneinander Wasserstoff oder C₁-C₁₂-Alkyl oder gemeinsam C₂-C₃-Alkylen oder C₂-C₃-Hydroxyalkylen darstellen; und
R₃ und R 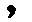 ₃ unabhängig voneinander Wasserstoff, Chlor, C₁-C₁₂-Alkyl oder C₁-C₁₂-Alkoxy bedeuten.

4. Verbindung der Formel I gemäß Anspruch 1, worin p 0 ist und
R₁ und R 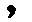 ₁ gleich sind und Wasserstoff, C₁-C₄-Alkoxy oder Chlor bedeuten;
R₂ und R 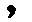 ₂ gleich sind und Wasserstoff oder C₁-C₄-Alkyl oder gemeinsam C₂-C₃-Alkylen oder C₂-C₃-Hydroxyalkylen darstellen; und
R₃ und R 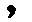 ₃ gleich sind und C₁-C₉-Alkyl oder C₁-C₄-Alkoxy bedeuten.

5. Verbindung der Formel I gemäß Anspruch 1, worin p 0 ist und
R₁ und R 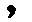 ₁ gleich sind und Wasserstoff oder Chlor bedeuten;
R₂ und R 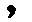 ₂ gleich sind und Wasserstoff oder Methyl bedeuten, oder R₂ und R 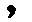 ₂ gemeinsam Ethylen oder 1,2-Dihydroxyethylen darstellen; und
R₃ und R 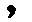 ₃ gleich sind und C₁-C₉-Alkyl bedeuten.

6. Zusammensetzung enthaltend
A) ein gegen Schädigung durch Licht, Sauerstoff und/oder Hitze empfindliches organisches Material und
B) als Stabilisator eine Verbindung der Formel I gemäß Anspruch 1.

7. Zusammensetzung gemäß Anspruch 6, enthaltend auf 100 Gew.-Teile der Komponente A 0,01 bis 15 Gew.-Teile der Komponente B.

8. Zusammensetzung gemäß Anspruch 6, enthaltend neben den Komponenten A und B noch andere Stabilisatoren und/oder sonstige Zusätze.

9. Zusammensetzung gemäß Anspruch 6, worin Komponente A ein organisches Polymer ist.

10. Zusammensetzung gemäß Anspruch 6, worin Komponente A
i) ein thermoplastisches Polymer ist, ausgewählt aus organischen Polymeren, die Heteroatome, insbesondere Stickstoff und/oder Sauerstoff, in der Hauptkette enthalten, Styrol-Copolymeren, Styrol-Pfropfcolymeren und Polymethylmethacrylaten (PMMA); oder
ii) ein Lackbindemittel ist.

11. Verfahren zum Stabilisieren von organischem Material gegen Schädigung durch Licht, Sauerstoff und/oder Hitze, dadurch gekennzeichnet, daß man diesem als Stabilisator eine Verbindung der Formel I gemäß Anspruch 1 zusetzt.

12. Verwendung von Verbindungen der Formel I gemäß Anspruch 1 zum Stabilisieren von organischem Material gegen Schädigung durch Licht, Sauerstoff und/oder Hitze.

## Claims

1. A compound of the formula I 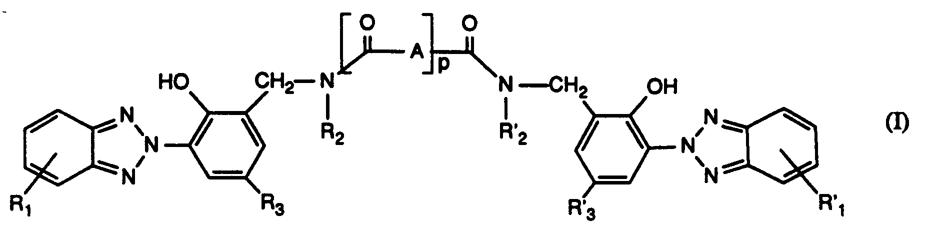
in which p is 0 or 1;
A is C₁-C₁₂alkylene;
R₁ and R'₁, independently of one another, are hydrogen, halogen, C₁-C₁₈alkyl, C₁-C₁₈alkoxy or -CN;
R₂ and R'₂, independently of one another, are hydrogen or C₁-C₁₈alkyl or together are C₂-C₁₂alkylene or C₂-C₁₂hydroxyalkylene; and
R₃ and R'₃, independently of one another, are hydrogen, halogen, C₁-C₁₈alkyl or C₁-C₁₈alkoxy.

2. A compound of the formula I according to claim 1, in which p is 0.

3. A compound of the formula I according to claim 1, in which
R₁ and R'₁, independently of one another, are hydrogen, halogen, C₁-C₁₂alkyl, C₁-C₁₂alkoxy or -CN;
R₂ and R'₂, independently of one another, are hydrogen or C₁-C₁₂alkyl or together are C₂-C₃alkylene or C₂-C₃hydroxyalkylene; and
R₃ and R'₃, independently of one another, are hydrogen, chlorine, C₁-C₁₂alkyl or C₁-C₁₂alkoxy.

4. A compound of the formula I according to claim 1, in which p is 0 and
R₁ and R'₁ are identical and are hydrogen, C₁-C₄alkoxy or chlorine;
R₂ and R'₂ are identical and are hydrogen or C₁-C₄alkyl or together are C₂-C₃alkylene or C₂-C₃hydroxyalkylene; and
R₃ and R'₃ are identical and are C₁-C₉alkyl or C₁-C₄alkoxy.

5. A compound of the formula I according to claim 1, in which p is 0 and
R₁ and R'₁ are identical and are hydrogen or chlorine;
R₂ and R'₂ are identical and are hydrogen or methyl, or R₂ and R'₂ together are ethylene or 1,2-dihydroxyethylene; and
R₃ and R'₃ identical and and are C₁-C₉alkyl.

6. A composition comprising
A) an organic material which is sensitive to damage by light, oxygen and/or heat, and
B) a compound of the formula I according to claim 1 as stabilizer.

7. A composition according to claim 6, comprising from 0.01 to 15 parts by weight of component B per 100 parts by weight of component A.

8. A composition according to claim 6, comprising other stabilizers and/or other additives in addition to components A and B.

9. A composition according to claim 6, in which component A is an organic polymer.

10. A composition according to claim 6, in which component A is
i) a thermoplastic polymer selected from organic polymers containing hetero atoms, in particular nitrogen and/or oxygen, in the main chain, styrene copolymers, styrene graft copolymers and polymethyl methacrylates (PMMA); or
ii) a paint binder.

11. A process for the stabilization of organic material against damage by light, oxygen and/or heat, which comprises adding a compound of the formula I according to claim 1 as stabilizer to the organic material.

12. The use of a compound of the formula I according to claim 1 for the stabilization of organic material against damage by light, oxygen and/or heat.

## Revendications

1. Composé de formule I 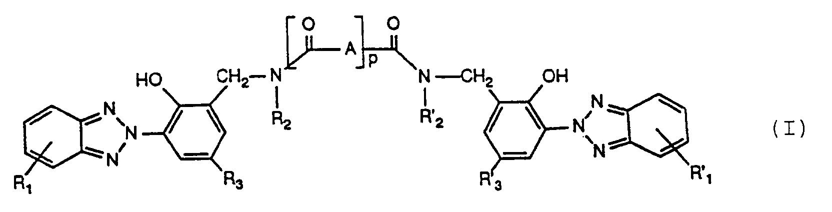
dans laquelle p vaut 0 ou 1 ;
A représente un groupe alkylène en C₁-C₁₂ ;
R₁ et R'₁, indépendamment l'un de l'autre, représentent un atome d'hydrogène, un atome d'halogène, des groupes alkyle en C₁-C₁₈, alcoxy en C₁-C₁₈ ou CN ;
R₂ et R'₂, indépendamment l'un de l'autre, représentent un atome d'hydrogène ou un groupe alkyle en C₁-C₁₈, ou ensemble représentent un groupe alkylène en C₂-C₁₂ ou hydroxyalkylène en C₂-C₁₂ ; et
R₃ et R'₃, indépendamment l'un de l'autre, représentent un atome d'hydrogène, un atome d'halogène, un groupe alkyle en C₁-C₁₈ ou alcoxy en C₁-C₁₈.

2. Composé de formule I selon la revendication 1, dans lequel p vaut 0.

3. Composé de formule I selon la revendication 1, où
R₁ et R'₁, indépendamment l'un de l'autre, représentent un atome d'hydrogène, un atome d'halogène, des groupes alkyle en C₁-C₁₂, alkoxy en C₁-C₁₂ ou CN ;
R₂ et R'₂, indépendamment l'un de l'autre, représentent un atome d'hydrogène ou un groupe alkyle en C₁-C₁₂, ou ensemble représentent un groupe alkylène en C₂-C₃ ou hydroxyalkylène en C₂-C₃ ; et
R₃ et R'₃, indépendamment l'un de l'autre, représentent un atome d'hydrogène, un atome de chlore, un groupe alkyle en C₁-C₁₂ ou alcoxy en C₁-C₁₂.

4. Composé de formule I selon la revendication 1, où p vaut 0 et
R₁ et R'₁ sont identiques et représentent un atome d'hydrogène, un groupe alcoxy en C₁-C₄ ou un atome de chlore ;
R₂ et R'₂ sont identiques et représentent un atome d'hydrogène ou un groupe alkyle en C₁-C₄ ou ensemble représentent un groupe alkylène en C₂-C₃ ou hydroxyalkylène en C₂-C₃ ; et
R₃ et R'₃ sont identiques et représentent un groupe alkyle en C₁-C₉ ou alcoxy en C₁-C₄.

5. Composé de formule I selon la revendication 1, où p vaut 0 et
R₁ et R'₁ sont identiques et représentent un atome d'hydrogène ou un atome de chlore ;
R₂ et R'₂ sont identiques et représentent un atome d'hydrogène ou le groupe méthyle, ou R₂ et R'₂ ensemble représentent l'éthylène ou le 1,2-dihydroxyéthylène ; et
R₃ et R'₃ sont identiques et représentent un groupe alkyle en C₁-C₉.

6. Composition contenant
A) une matière organique sensible à la dégradation par la lumière, par l'oxygène et/ou par la chaleur et
B) un composé de formule I selon la revendication 1 en tant que stabilisant.

7. Composition selon la revendication 6, contenant pour 100 parties en poids du composant A de 0,01 à 15 % en poids du composant B.

8. Composition selon la revendication 6, contenant outre les composants A et B encore autres stabilisants et/ou autres additifs.

9. Composition selon la revendication 6, où le composant A est un polymère organique.

10. Composition selon la revendication 6, où le composant A
i) est un polymère thermoplastique pris parmi les polymères organiques qui comportent des hétéroatomes, plus particulièrement des atomes d'azote et/ou d'oxygène dans la chaîne principale, des copolymères de styrène, des copolymères greffés de styrène et des poly(méthacrylates de méthyle) (PMMA) ; ou
ii) est un liant de vernis.

11. Procédé pour la stabilisation des matières organiques contre la dégradation induite par la lumière, par l'oxygène et/ou par la chaleur, caractérisé en ce que l'on ajoute à celles-ci comme stabilisant un composé de formule I selon la revendication 1.

12. Utilisation de composés de formule I selon la revendication 1, pour la stabilisation des matières organiques contre la dégradation induite par la lumière, par l'oxygène et/ou par la chaleur.
